# EUROPEAN PATENT SPECIFICATION

(11) **EP 1 651 795 B1**
(45) Date of publication and mention of the grant of the patent: **30.05.2012**
(21) Application number: 04756143.6
(22) Date of filing: 25.06.2004
(51) Int. Cl.: C23C 18/28, C23C 18/44, C23C 18/16

(54) **ELECTROLESS DEPOSITION METHODS AND SYSTEMS**
VERFAHREN UND SYSTEM ZUR STROMLOSEN ABSCHEIDUNG
PROCEDES ET SYSTEMES DE DEPOT AUTOCATALYTIQUES

(30) Priority: 11.07.2003 US 618049
(43) Date of publication of application: 03.05.2006
(73) Proprietor: HEWLETT-PACKARD DEVELOPMENT COMPANY, L.P., Houston, TX 77070 (US)
(72) Inventor: MARDILOVICH, Peter, Corvallis, OR 97330 (US); HERMAN, Gregory S., Albany, OR 97321 (US); PUNSALAN, David, Eugene, OR 97401 (US); BERHANE, Samson, Corvallis, OR 97330-4239 (US)
(74) Representative: Lawman, Matthew John Mitchell
(86) International application number: PCT/US2004/020498
(87) International publication number: WO 2005/007930

(56) References cited:
- EP-A- 0 346 954
- EP-A- 0 616 053
- WO-A-97/22733
- WO-A-02/099162
- WO-A-03/049515
- US-A- 3 723 158
- US-A- 4 091 128
- US-A- 4 150 171

## Description

### FIELD OF THE INVENTION

The present invention relates generally to printing of metals using an electroless process.

### BACKGROUND OF THE INVENTION

Computer printer technology has evolved to a point where very high-resolution images can be transferred to various types of media. Ink-jet printing involves the placement of small drops of a fluid ink onto a media surface in response to a digital signal. Common ink-jet printing methods include thermal ink-jet and piezoelectric ink-jet technologies, although other ink-jet methods are known. Typically, the fluid ink is placed or jetted onto the surface without physical contact between the printing device and the surface. There are several reasons that ink-jet printing has become a popular way of recording images on various media surfaces, particularly paper. Some of these reasons include low printer noise, capability of high-speed recording, and multi-color recording. Additionally, these advantages can be obtained at a relatively low price to consumers.

Production of circuits and conductive traces has been accomplished in many different ways. Further, various methods for manufacturing printed circuit boards are known. Typical methods for manufacturing printed circuits include print and etch, screen printing, and photoresist methods, e.g., applying photoresist, exposing, and developing. Frequently, these methods involve considerable capital cost and production time. In recent years, ink-jet technologies have been used to form circuitry. These ink-jet technologies include a variety of methods which have been met with varying degrees of success.
Some of these methods involve ink-jetting of a precursor material which aids in deposition of conductive metals. Other methods involve printing of conductive inks onto a substrate. Each of these methods has disadvantages which limit their effectiveness, such as expense, reliability, performance, and complexity.

International patent publication no. WO02/099162 describes a method of preparing a substrate material such that it is capable of sponsoring a catalytic reaction over a pre-determined area of its surface. A catalytic material may be laid down onto the substrate by inkjet printing.

US patent no. US-A-4150171 describes electroless plating of a substrate with aqueous solutions containing stannous and copper ions.

International patent publication no. WO03/049515 describes depositing a material onto a substrate using an inkjet printer. For example, a PCB may be printed by an inkjet printer by printing a metal salt and a reducing agent.

US patent no.US-A-3723158 describes wet chemical methods of providing nonconductive transparent substrates with a transparent coating having a metallic luster and good uniformity.

US patent no.US-A-4091128 describes a method for preparing an electroless gold plating bath.

European patent no. EP-A-0616053 describes a process for applying a metal coating to a nonconductive substrate without an electroless coating.

Investigations continue into developing improved circuit fabrication techniques and compositions for use with ink-jet technologies.

### SUMMARY OF THE INVENTION

It has been recognized that it would be advantageous to develop inexpensive and simple methods for forming metal patterns which can be conductive, such as interconnects.

One aspect of the present invention includes a method of forming metal patterns on a substrate using ink-jet technology and ink-jettable compositions according to claim 1. An electroless active layer can be formed over at least a portion of the substrate using any number of techniques. An ink-jettable metal composition can be jetted on the electroless active layer in a predetermined pattern. The ink-jettable metal composition can include a metal salt and an optional liquid vehicle. An ink-jettable reducing agent composition can also be ink-jetted on the pattern. The reducing agent composition includes a reducing agent and an optional liquid vehicle, wherein the reducing agent can react with the metal salt to form a reduced conductive metal on the pattern.

Another aspect of the present invention includes a system according to claim 9 for forming metal patterns on a substrate using ink-jettable compositions having a first printhead, a second printhead, and an activation system. The first printhead can include a first firing chamber reservoir containing an ink-jettable metal composition including a metal salt. The second printhead can have a second firing chamber reservoir containing an ink-jettable reducing agent composition including a reducing agent, which can be overprinted or underprinted on a substrate with respect to the metal composition.

In another embodiment, a system for forming metal patterns on a substrate can comprise means for forming an electroless active layer on the substrate; means for printing an ink-jettable metal composition on at least a portion of the electroless active layer, wherein the ink-jettable metal composition includes a metal salt; and means for printing an ink-jettable reducing agent composition on at least a portion of the electroless active layer, wherein the ink-jettable reducing agent composition includes a reducing agent. Upon printing the metal composition and the reducing agent composition, contact occurs forming a reduced metal.

Additional features and advantages of the invention will be apparent from the detailed description which illustrates, by way of example, features of the invention.

### DETAILED DESCRIPTION OF SEVERAL EMBODIMENTS

Before particular embodiments of the present invention are disclosed and described, it is to be understood that this invention is not limited to the particular process and materials disclosed herein as such may vary to some degree. It is also to be understood that the terminology used herein is used for the purpose of describing particular embodiments only and is not intended to be limiting, as the scope of the present invention will be defined only by the appended claims and equivalents thereof.

In describing and claiming the present invention, the following terminology will be used.

The singular forms "a," "an," and "the" include plural referents unless the context clearly dictates otherwise. Thus, for example, reference to "a reducing agent" includes reference to one or more of such materials.

As used herein, "liquid vehicle" is defined to include liquid compositions that can be used to carry a metal salt composition, a reducing agent, and/or optionally, colorants or other compositions to a substrate. Liquid vehicles are well known in the art, and a wide variety of liquid vehicles may be used in accordance with embodiments of the present invention. Such liquid vehicles may include a mixture of a variety of different agents, including without limitation, surfactants, solvents, co-solvents, buffers, biocides, viscosity modifiers, stabilizing agents, complexing agents, and water. Though a variety of agents are described that can be used, the liquid vehicle, in some embodiments, can be simply a single liquid component, such as water. Though liquid vehicles are described herein in some detail, it is to be understood that the amount of liquid vehicle can vary over a wide range, and is optional. For example, in some embodiments, the metal salt and/or reducing agent can be configured to be jetted from ink-jet architecture without the use of additional liquid vehicle, e.g., the metal salt and/or reducing agent can be acquired in dry form or aqueous solution. Such compositions containing small amounts of liquid vehicle can be used in piezoelectric ink-jet printing systems, for example. Alternatively, thermal ink-jet ink systems can be used, and appropriate amounts of liquid vehicle can be used, as would be known by one skilled in the art after considering the present disclosure. An example where a liquid vehicle can be added is with respect to embodiments wherein it is desired to alter the viscosity, surface tension, pH, or the like, of the ink-jettable metal composition and/or the reducing agent composition. Several considerations in selecting the amount of liquid vehicle include those related to nucleation such as heat capacity, heat of vaporization, critical nucleation temperature, diffusivity, and the like. For example, aqueous liquid vehicles often have at least 5% to 10% water to provide sufficient nucleation in thermal ink-jet systems.

As used herein, "solvated" refers to when a solute is dissolved into a solvent. A compound that is solvated indicates that at least a portion of the compound is dissolved into solution and does not necessarily indicate that all of the solute molecules are in solution.

As used herein, "electroless deposition" refers to a chemical deposition of a metal as opposed to electrodeposition. Typically, electroless deposition processes involve chemical baths which can be acidic, basic, or neutral; and contain metal ions, which in the presence of a reducing agent, can be reduced to the metal. However, other such processes known to those skilled in the art are considered within the scope of the present invention.

As used herein, "ink-jetting" refers to the well known process of depositing liquids using ink-jet architecture, and is in no way limited to depositing inks or ink-containing compositions. Thus, although some embodiments of the present invention may include inks, this is not required. Similarly, ink-jetting of materials "on" a substrate can include direct contact of such material with the substrate or can indicate that the material is printed in contact with a separate material or layer which is in direct or indirect contact with the substrate.

When referring to a "first printhead" and a "second printhead," it is not to be inferred that a single common printhead is excluded. The first printhead and the second printhead can be a common printhead. For example, a single orifice plate can be used to separately jet a metal composition and a reducing agent composition. In this case, the first and second printhead are the same, and each composition can be housed by separate firing chambers on the common orifice plate, for example.

Concentrations, amounts, and other numerical data may be presented herein in a range format. It is to be understood that such range format is used merely for convenience and brevity and should be interpreted flexibly to include not only the numerical values explicitly recited as the limits of the range, but also to include all the individual numerical values or sub-ranges encompassed within that range as if each numerical value and sub-range is explicitly recited. For example, a weight range of 1% to 20% should be interpreted to include not only the explicitly recited concentration limits of 1% to 20%, but also to include individual concentrations such as 2%, 3%, 4%, and sub-ranges such as 5% to 15%, 10% to 20%, etc.

In accordance with the present invention, metal patterns can be formed on a substrate using ink-jet methods in conjunction with electroless metal deposition systems.

### Electroless Active Layer

An electroless active layer can be formed over at least a portion of a substrate. Means for such applications are included herein. The electroless active layer can be metal deposited on the substrate surface, which acts as a seed or electroless initiator for electroless metal deposition. The electroless initiator can be deposited using physical vapor deposition, metal catalyst salt solutions, or any known metal deposition process which can form an activated surface suitable for deposition using electroless processes. If metal catalyst salt solutions are used, such solutions can be printed using ink-jet or other known printing methods, or prepared in a bath into which the substrate is immersed. Typical electroless initiators which can be used include, without limitation, palladium, aluminum protected copper, silver, and mixtures thereof. Experiments have shown that successive exposure of the substrate to tin and palladium chloride solutions, respectively, provide a good balance between surface adhesion and uniform distribution of palladium seed, see Mardilovich et al., "Defect-Free Palladium Membranes on Porous Stainless-Steel Support", AIChE Journal, vol. 44, no.2 (1998). Suitable palladium salts can include palladium salts with chloride, bromide, iodide, acetate, trifluoroacetate, acetylacetonate, carbonate, perchlorate, nitrate, sulfate, and/or oxide. Other metal deposition techniques and compositions are known to those skilled in the art and can be chosen, based on the considerations outlined herein, to produce an activated substrate surface suitable for electroless deposition. Those skilled in the art will recognize that some suitable deposition methods may require the use of masks, etching, plasma treatment, and/or other additional steps.

In an embodiment of the present invention, the electroless active layer is formed by marring the surface of the substrate in lieu of, or in addition to, depositing a metal seed. Marring of the substrate can be accomplished by increasing the surface roughness of at least the areas where deposition of metal during the electroless process is desired. The surface roughness can be increased by physically scratching, cracking, or etching the surface (e.g. using HF on a glass substrate, etc.), or by other known methods which involve marring of the surface of the substrate. Physical scratching of the substrate can be accomplished using a sharp tool or needle to achieve a specific pattern or may involve the use of abradants such as diamond, alumina, or other abrasives. Likewise, etching of the substrate can be accomplished using known chemical etchants that are appropriate for a particular substrate. For example, etching can be accomplished using oxygen plasma, a strong acid such as HCl, a mixture H₂O₂ with HNO₃, or other similar compositions which would react with a particular substrate to increase surface roughness. The surface roughness for electroless deposition of metals can vary somewhat depending on the substrate; however a surface roughness of greater than from 10 nm to several micrometers can be sufficient for purposes of the present invention.

The surface sites that have undergone marring can then act as a nucleation or active site for deposition of metal from an electroless solution. Such a rough surface can act as a seed for electroless deposition in a similar manner to the deposition of metal seed, and is often sufficient to prevent substantial electroless reduction of metal in solution and encourage electroless deposition of metal on the substrate with sufficient adhesion to avoid peeling therefrom.

The electroless active layer is formed on portions of the substrate thereof which correspond to a predetermined pattern. In one embodiment, the entire surface of the substrate can be activated, such as by immersing the substrate in a bath as discussed above. Alternatively, the electroless active layer can be formed over discrete areas using any suitable printing technique such as, but not limited to, ink-jet printing, screen printing, gravure printing, offset printing, photolithographic printing, and the like. Similarly, discrete areas can be formed by masking portions of the substrate, such as by forming a thin hydrophobic layer on the substrate, which leaves unmasked the areas where activation is desired. The exposed areas can then be activated using the above described physical or chemical methods, or other known methods. Several hydrophobic materials suitable for use in the present invention, include without limitation, alkyltrichlorosilanes; fluorinated polymers such as TEFLON or FLUOROPEL (available from Cytonix Corporation, Beltsville, MD); and the like.

The electroless active layer can be formed in a continuous pattern which corresponds to a desired predetermined pattern. However, it should be noted that the electroless active layer can also be formed in a non-continuous pattern, wherein individual seeds of metal or activated areas of pattern provide for the deposition of a conductive metal. The non-continuous pattern can be formed of a series of dots which are sufficiently close such that electroless deposition of a conductive metal on the activated areas will ultimately connect proximate areas to form the desired predetermined pattern. In the absence of impurities or other non-homogenous conditions, electroless deposition generally occurs uniformly in all directions. Thus, if a continuous trace is desired, the distance between each dot can be less than the thickness of the deposited film. The predetermined pattern can be almost any pattern, but is most often a conductive pattern useful in the electronics industry such as in the form of a circuit pattern, antenna pattern, or the like.

A variety of methods can be utilized to produce non-continuous patterns. An electroless initiator can be deposited by exposing the substrate surface to a bath, as described above, such that deposition and nucleation of the metal only reaches the desired dot size. For example, the exposure time can be shortened, e.g. less than several seconds and even several milliseconds. Alternatively, the concentration of metal salt can be reduced such that reaction times are extended. In another alternative CVD or PVD deposition of an electroless initiator catalyst can be performed to form proximate catalyst seeds. Although the placement of such seeds will be somewhat random, the deposition can be limited to the time it takes to form seeds having the desired size, e.g. 0.5 nm to 100 nm, although sizes up to 1.0 µm can be used. Similarly, CVD or PVD deposition can be directed on the predetermined pattern by masking portions of the substrate with a removable material, e.g. polymer, or a CVD or PVD inactive material which does not allow deposition of a particular metal on the substrate.

### Electroless Metal Salt Compositions

In accordance with systems and methods of the present invention, electroless compositions can be ink-jetted onto a substrate in at least two separate compositions including a metal salt and a reducing agent. Specific electroless plating compositions and conditions can be chosen by those skilled in the art to achieve various plating rates, thicknesses, and conductivities. Any metal composition can be used in the present invention which is capable of being electrolessly deposited. A wide variety of electroless systems are known, and several exemplary systems are discussed below. Electroless deposition systems which result in autocatalytic plating of metal using various electroless systems can include, metal salts such as Pd(NH₃)₄Cl₂, Pd(NH₃)₄Cl₂·H₂O, Pd(NH₃)₄(NO₃)₂, Pd(NH₃)₄(NO₃)₂·H₂O, PdCl₂, AgNO₃, Cu(NO₃)₂, CuSO₄, CuSO₄·5H₂O, KAu(CN)₂, Na₃Au(S₂O₃)₂, NiSO₄, cobalt salts, and mixtures or hydrates thereof.

In accordance with the present invention, a metal composition can be printed in a pattern on the electroless active layer. Means for such applications are included herein. The metal composition can include a metal salt such as those listed above. In one aspect of the present invention, the metal composition includes a metal salt, the metal being selected from the group consisting of palladium, copper, silver, gold, nickel, cobalt, platinum, rhodium, and mixtures or alloys thereof. Further, the metal composition can include salts of a magnetic alloy such as Co-Fe-B, Co-Ni-P, Co-Ni-Fe-B, Ni-Co, and mixtures thereof. Such magnetic alloys are typically printed on rigid substrates to avoid variations in magnetic properties under mechanical stress. In one detailed aspect of the present invention, the metal salt can be a member selected from the group consisting of Pd(NH₃)₄Cl₂, PdCl₂, Pd(NH₃)₄(NO₃)₂, AgNO₃, Cu(NO₃)₂, and mixtures thereof. In another detailed aspect of the present invention, the metal composition can include a metal salt of palladium such as Pd(NH₃)₄Cl₂. The metal composition may optionally include other components such as complexing agents such as ethylenediaminetetraacetic acid (EDTA), di-sodium EDTA, sodium acetate, phenylenediamine, sodium potassium tartrate, nitrilotriacetic acid, and pH modifiers such as ammonium hydroxide, sodium hydroxide, potassium hydroxide, sodium carbonate, and potassium carbonate. In one embodiment, the metal salts of the present invention can be solvated in a liquid vehicle such that the ink-jettable compositions of the present invention include from 0.01 wt% to 5 wt% of metal salt, such as 0.04 to 1 wt%.

### Electroless Reducing Agent

Upon ink-jetting the metal salt composition onto a substrate, the metal ions of the metal salt generally retains its oxidation state, and even upon drying, will not be reduced to its metallic form. In accordance with the present invention, a reducing agent composition can be ink-jetted by underprinting or overprinting with respect to the metal composition, and means for such applications are included herein. The reducing agent composition can include a reducing agent in an optional liquid vehicle, wherein the reducing agent reacts with the metal salt to form a reduced metal on a pattern. It should be understood that the reducing agent compositions of the present invention can be ink-jetted on the substrate prior to, subsequent to, or simultaneously with ink-jetting of the metal salt composition. A variety of reducing agents are suitable for use in the present invention, and may include any composition which reacts with the metal salt to form a reduced metal. Those skilled in the art will recognize that specific reducing agents may effectively reduce a number of different metal salts, or be limited to use with specific metal salts. Several reducing agents which are suitable for use in the present invention include, but are not limited to, formaldehyde, hydrazine, sodium hypophosphite, sodium boron hydride, dimethylaminoboran, sodium L-ascorbic acid, and mixtures or hydrates thereof. Other suitable reducing agents include formalin, dimethylamine borane, alkali metal borohydrides, triethylaminoborane, borane-t-butylamine, borane pyridine, oxalic acid, formic acid, esters of formic acid, formic acid derivatives, substituted and non-substituted amides such as formamide and N, N-dimethyl formamide, salts of formic acid such as sodium formate, activated formic acids such as orthoformic acid, alkali or alkaline earth sulfites, and mixtures thereof. In one embodiment of the present invention, the reducing agent can be hydrazine. Specific electroless metal salt and reducing agent systems are known to those skilled in the art, however several common systems include sodium hypophosphite for Ni and Co salts; sodium borohydride for Ni and Au salts; hydrazine for Ni, Au, Pd, and Ag salts; formaldehyde for Cu salts; and dimethylamineborane (or other substituted amine boranes) for Ni, Co, Au, Cu, and Ag salts.

In accordance with the methods and systems of the present invention, the reducing agent composition can be applied onto at least a portion of the predetermined pattern. In some applications, it may be desirable to only apply reducing agent to portions of the metal salt and/or pattern printed on the substrate. Further, portions of the metal salt can be reduced to metallic metal followed by further steps which react or utilize the metal, but do not affect the metal salt not having reducing agent applied thereto. At a later step, the remaining metal salt can then be reduced by applying reducing agent thereto. In an additional alternative embodiment, the reducing agent can be applied to the substrate prior to printing the metal salt thereon. In other embodiments, the reducing agent can be applied to the entire substrate or only to the areas where the metal salt is applied.

The concentration of metal salt and reducing agent will also affect the rate and quality of the deposited metal. For example, a highly concentrated solution of either component will, for most electroless systems, result in the nearly spontaneous metal reduction/precipitation, e.g. less than about a second to consume the limiting reagent. In such cases, the metal is not adhered to the active layer in a continuous film, but rather metal nanoparticles primarily in the solution.

The reducing agent composition can further include solvents, cosolvents, surfactants, biocides, buffers, viscosity modifiers, sequestering agents, colorants, stabilizing agents, humectants, water, and mixtures thereof as discussed below. In one embodiment, the reducing agent and/or metal salt composition can include pigment and/or dye colorants which can be used for aesthetic purposes or to identify specific traces. Typical ink-jettable reducing agent compositions can include from 0.01 % to 5 % by weight of reducing agent, such as 0.02 % to 2 % by weight. The amount of reducing agent applied to the pattern on the substrate is most often sufficient to provide stoichiometric or excess amounts of reducing agent.

Once the reducing agent is applied to the desired portions of the predetermined pattern, heat can optionally be applied. Frequently, the reaction kinetics of the deposition reaction are such that temperatures of from 20°C to 120 °C are desirable. Thus, in some embodiments of the present invention, heat can be supplied by either heating the substrate and/or ink-jet compositions. Heating the substrate prior to ink-jetting compositions thereon can further enhance the ability to obtain finer linewidths, as the volatile components of the jetted compositions can evaporate thus increasing viscosity. Alternatively, heat can optionally be applied subsequent to ink-jetting of metal salt and/or reducing agent. The amount of heat generated and/or applied to the predetermined pattern can be sufficient to cause reaction between the reducing agent and the metal salt without substantially altering or damaging the substrate. If necessary, heat can be applied to the predetermined pattern using almost any heat source. Any number of heating apparatuses can be used such as heater bars, heat lamps, heating plates, forced heated air, or other known heat sources.

Although conditions may vary depending on the electroless system used, electroless plating temperatures can range from 15 °C to 120 °C, and reaction times can range from 1 minute to 16 hours, but are more typically from 10 to 45 minutes. Electroless deposition depths can range from 0.01 to 100 µm, with 0.1 to 20 µm being a common depth for many applications. Almost any depth can be achieved by repeating ink-jetting of metal composition and reducing agent steps until a desired depth is reached. Alternatively, ink-jetting of hydrophilic metal salt and reducing agent compositions on a hydrophobic surface, such as those discussed above, allows for an increased thickness of printed compositions and a resulting increase in deposited metal depths per cycle of ink-jetting metal salt and reducing agent.

### Additional Considerations

The placement of metal salt compositions and reducing agent compositions in separate ink-jet printheads allows for significant latitude in formulating ink-jettable compositions which are tailored to various ink-jet pens, including thermal, piezoelectric, sonic impulse, or other known ink-jet printing systems. Additionally, in some embodiments it may be desirable to print either the metal composition or reducing agent with a colored ink composition, such as by simultaneous printing, overprinting, or underprinting with respect to the electroless ink-jet compositions. Frequently, such color ink compositions involve pH driven bleed control mechanisms. The ink-jettable compositions of the present invention can be easily incorporated into such systems such that various colorants can be added directly to the metal salt compositions, reducing agent compositions, or placed in its own composition to be printed separately. Further, specific printhead and substrate materials may be more or less resistant to corrosion when exposed to compositions at various pH levels. In addition to a wide pH range and increased stability, the electroless metal salts of the present invention are typically present in solution. Further, the method of the present invention decreases some of the difficulties in stabilizing electroless systems since the metal salt and reducing agent are not stored together in the printing system. As a result, kogation and clogging problems are reduced and storage life can be increased. In addition, various additives such as stabilizers and buffer solutions are also often unnecessary. Similarly, additional irradiation or other processing steps can be unnecessary in the methods of the present invention.

The ink-jettable compositions of the present invention, e. g. metal salt composition and reducing agent composition, can include a variety of components such as those typically used in ink-jet liquid vehicles, such as, but not limited to solvents, cosolvents, surfactants, biocides, buffers, viscosity modifiers, sequestering agents, colorants, stabilizing agents, humectants, water, and mixtures thereof. Typically the ink-jettable compositions for use in thermal ink-jet systems can have a viscosity of from 0.8 x 10⁻³ Pa.s (0.8 cP) to 8 x 10⁻³ Pa.s (8 cP), and in some cases, may be up to 15 x 10⁻³ Pa.s (15 cP). Similarly, inkjettable compositions for use in piezoelectric ink-jet systems can have a viscosity of from 2 x 10⁻³ Pa.s (2 cP) to 15 x 10⁻³ Pa.s (15 cP), and in some cases, may be up to 30 x 10⁻³ Pa.s (30 cP). Although a variety of viscosity modifiers can be used, several common compounds include 2-pyrrolidone, isopropyl alcohol, glycerol, and the like. Similarly, the surface tension of ink- jettable compositions used in thermal ink-jet systems can range from 2.5 N/m² (25 dyne/cm²) to 7.5 N/m² (75 dyne/cm²), and in some embodiments, can be from about 3 to 5.5 N/m² (30 to about 55 dyne/cm²). The surface tension can be adjusted using compounds such as isopropyl alcohol, ethanol, methanol, glycerol, water, and the like. In one aspect of the present invention, the liquid vehicle can comprise from 70% to 98% by weight of the ink-jettable composition. Various techniques can be used to modify the viscosity or other jetting properties of the ink-jettable compositions herein. For example, heat can be used to liquefy material, increase solubility of the desired material, or reduce viscosity such that it becomes ink-jettable. Those skilled in the art will recognize that the above discussion is primarily focused on thermal ink-jet systems, as piezoelectric ink-jet systems involve less restrictive considerations. For example, thermal ink-jet systems are typically operated at temperatures below 80 °C, while piezoelectric ink-jet systems can be operated at temperatures of up to 150 °C. In light of the present disclosure, those skilled in the art will recognize which components can be included in the liquid vehicle in order to ink-jet compositions of the present invention from thermal, piezoelectric, or other ink-jet systems.

As described, cosolvents can be included in the ink-jettable compositions of the present invention. Suitable cosolvents for use in the present invention include water soluble organic cosolvents, but are not limited to, aliphatic alcohols, aromatic alcohols, diols, glycol ethers, poly (glycol) ethers, lactams, formamides, acetamides, long chain alcohols, ethylene glycol, propylene glycol, diethylene glycols, triethylene glycols, glycerine, dipropylene glycols, glycol butyl ethers, polyethylene glycols, polypropylene glycols, amides, ethers, carboxylic acids, esters, organosulfoxides, sulfones, alcohol derivatives, carbitol, butyl carbitol, cellosolve, ether derivatives, amino alcohols, and ketones. For example, cosolvents can include primary aliphatic alcohols of 30 carbons or less, primary aromatic alcohols of 30 carbons or less, secondary aliphatic alcohols of 30 carbons or less, secondary aromatic alcohols of 30 carbons or less, 1, 2-diols of 30 carbons or less, 1, 3-diols of 30 carbons or less, 1, 5-diols of 30 carbons or less, ethylene glycol alkyl ethers, propylene glycol alkyl ethers, poly (ethylene glycol) alkyl ethers, higher homologs of poly(ethylene glycol) alkyl ethers, poly(propylene glycol) alkyl ethers, higher homologs of poly(propylene glycol) alkyl ethers, lactams, substituted formamides, unsubstituted formamides, substituted acetamides, and unsubstituted acetamides. Specific examples of cosolvents that can be used in the practice of this invention include, but are not limited to, 1, 5-pentanediol, 2-pyrrolidone, 2-ethyl-2-hydroxymethyl-1, 3-propanediol, diethylene glycol, 3-methoxybutanol, and 1,3-dimethyl-2-imidazolidinone. Cosolvents can be added to reduce the rate of evaporation of water in the composition to minimize clogging or other properties of the composition such as viscosity, pH, surface tension, and print quality. The cosolvent concentration can range from about 0% to about 50% by weight, and in one embodiment can be from about 15% to about 30% by weight. Multiple cosolvents can also be used, wherein each cosolvent can be typically present at from about 2% to about 10% by weight of the ink-jettable composition.

Various buffering agents can also be optionally used in the ink-jettable compositions of the present invention. Typical buffering agents include such pH control solutions as hydroxides of alkali metals and amines, such as lithium hydroxide, sodium hydroxide, and potassium hydroxide; and other basic or acidic components. If used, buffering agents typically comprise less than 10% by weight of the ink-jettable composition.

In one aspect of the present invention, the ink-jettable compositions can optionally contain surfactants. Such components can be used and may include standard water-soluble surfactants such as alkyl polyethylene oxides, alkyl phenyl polyethylene oxides, polyethylene oxide (PEO) block copolymers, acetylenic PEO, PEO esters, PEO amines, PEO amides, and dimethicone copolyols. If used, surfactants can be from 0. 01 % to 10% by weight of the ink-jettable composition.

As mentioned above, in accordance with the present invention, temperatures used in electroless deposition are frequently below 120° C, and in many cases, can be from 20 °C to 90 °C. For example, the electroless deposition of palladium using Pd(NH₃)₄Cl₂ and hydrazine can occur at temperatures as low as 40 °C, although slowly. At temperatures below 120 °C, most substrate materials are not adversely affected. Thus, substrate materials suitable for use in the present invention can include, without limitation, ceramics, polymers, cellulose, glass, silicon, organic substrates, metal oxides, and mixtures or composites thereof. For example, the compositions of the present invention can be printed on a standard silicon substrate, polyethylene terephthalate (available from E. I. du Pont de Nemours and Company as MYLAR), polyimides (available from E. I. du Pont de Nemours and Company as KAPTON), epoxy resins, phenolic resins, polyester resins, aluminum nitride, glass, alumina ceramic, or even certain papers in some low temperature applications. Although the above mentioned substrates are suitable, almost any non-conductive material or flexible or non-flexible dielectric material can be used as the substrate in the present invention. In addition, the methods of the present invention can be applied to substrates having previously formed electronic circuits and/or devices thereon using any known method.

The circuits produced in accordance with the principles of the present invention can form a wide variety of electronic devices and the resolution and complexity of such pathways are only limited by the ink-jet printing technology. Circuit patterns can include, for example, complex circuits, single traces, antennae, or even multilayered circuits. Patterns formed using the ink-jettable composition of the present invention can have a linewidth of from about 5 micrometers to any practical width. Generally, a width of from 2 to 10 millimeters is the widest practical width; however, wider patterns could be formed depending on the application. In one aspect of the present invention, linewidths can be from 80 µm to 500 µm. Those skilled in the art will recognize, upon review of the present disclosure, that linewidths can be affected by the rate of evaporation and the porosity of specific substrates. For example, as mentioned herein, some embodiments involve ink-jetting on a heated substrate. In such embodiments, the ink-jetted compositions can experience a decrease in viscosity as the composition heats, followed by a decrease in viscosity due to water and/or other solvents evaporating. These competing effects can be adjusted by those skilled in the art to achieve specific linewidths and edge acuities.

Similarly, the predetermined pattern can have varying depths as measured from the substrate to an upper surface of the conductive pathway. The depth of the electrodeposited metal can be easily controlled by the ink-jetting process during printing of the electroless compositions. Namely, the depth of the conductive metal is governed by the length of time the surface is exposed to the electroless solution, particular solution, viscosity, and concentrations used. Additionally, the depth can be increased by repeating the ink-jetting of metal salt composition and reducing agent composition as many times as necessary to achieve the predetermined depth. The predetermined depth can range from 0.01 µm to 100 µm, although depths of from 0.1 µm to 20 µm are desirable for most electronic devices.

In an alternative embodiment, metal salt compositions and reducing agent concentrations having concentrated, e.g. above 2 to 3 wt% depending on the electroless system, can be ink-jetted in slightly offset areas. Specifically, a pattern printed from the metal salt composition can have reducing agent printed adjacent thereto such that the pattern or line of reducing agent will overlap just at the edges of the printed areas. Deposition of metal can begin in this overlap area and spread in both directions due to the diffusion of metal salt and reducing agent through the deposited compositions. Typically, in the aforementioned embodiment, the metal film will be thicker in the middle and the width of the metal film can be smaller than the overall width of jetted metal salt and reducing agent. Control of the evaporation rate of the lines forming the overlap region is one method that can be used to control/limit the width of the resulting metallic trace/line. Control of the evaporation rates can be accomplished by adjusting variables such as temperature, viscosity, and concentrations of volatile components in the compositions.

In another alternative embodiment, the metal salt and reducing agent can be deposited simultaneously with spatial misalignment such that adjacent drops of metal salt and reducing agent are touching each other but not overlapping. At point of contact between adjacent drops the deposition reaction will begin. The metal deposition will spread out from the point of contact toward outer edges of the drops. In any of the above embodiments, impurities in the deposited compositions can be minimized to achieve conductivities which approach the bulk conductivity of the deposited metal. Thus, reducing agents and metal salt counter ions can be used which minimize deleterious by-products and impurities in the deposited metal.

Using the methods described herein, almost any known predetermined pattern can be defined on the electroless active layer, such as, gates, transistors, diodes, resistors, inductors, capacitors, traces, magnets, other circuit elements, and decorational patterns. The present invention allows the production of a wide variety of devices in a short period of time and with minimal preparation, which normally accompanies standard lithography techniques of preparing a mask, deposition, etching, etc. Thus, prototypes of complex patterns can be tested and adjusted without time consuming lithography steps.

In one aspect of the present invention, the predetermined pattern can be printed in multiple layers to form three dimensional structures. For example, a first layer containing a predetermined pattern can be produced by printing the electroless compositions and forming a conductive pathway using any of the above described embodiments. A layer of insulating or semi-conducting material of a polymeric resin, organic resin, doped ceramic, semiconductor, or the like, can be formed over the first layer. Most often, the insulating layer can be discontinuous having conduits or holes in which additional conductive metal can be placed. These holes can be formed after the deposition of the insulating material using standard lithography technologies. A conductive metal can then be printed using the methods of the present invention, or otherwise deposited. Alternatively, the holes can be formed by printing a material prior to forming the insulating material which prevents the insulating material or semi-conducting material from adhering to the predetermined pattern at specific locations. A second layer of conductive circuits or pathways can then be printed on the insulating material using the above principles. This process can be repeated as many times as needed to form a desired multilayer circuit.

### System Incorporating Ink-Jettable Compositions

A variety of techniques can be used to form metal patterns on various substrates. In one embodiment of the present invention, a system for forming metal patterns includes an activation system, a first printhead, and a second printhead. An ink-jet printer, for example, can be used to propel ink-jet compositions onto substrates using resistive heating elements or piezoelectric elements for propelling the composition through an overlying orifice plate. The ink-jet compositions can be stored in a reservoir and the composition can travel through a set of channels toward the orifice plate. In connection with the present invention, the first printhead can have a first firing chamber reservoir containing an ink-jettable metal composition. The ink-jettable metal composition can include a metal salt, as previously described. The second printhead can have a second firing chamber reservoir containing an ink-jettable reducing agent composition. This ink-jettable reducing agent composition can include a reducing agent and an optional liquid vehicle, as previously discussed.

The system of the present invention utilises the marring of the substrate as electroless activation techniques as described above. Preferably the activation system can be a tip configured for marring the surface in a predetermined pattern. The tip can be any rigid member which will cause marring of the substrate upon contact therewith.

In an alternative embodiment, an additional printhead can have a firing chamber reservoir containing a colored ink-jettable composition or a rinsing solution such as water. Thus, for example, between deposition of layers of metal or after the electroless deposition process is complete, the substrate can be rinsed. Similarly, various marks or images can be printed using a colored ink without removing the substrate from the ink-jet system in a single process.

It will be understood that the first and second printhead can be present on a common orifice plate as a single printhead assembly, or on separate orifice plates. Typically, a common orifice plate configuration would benefit from the use of non-reactive liquid vehicles in order to reduce clogging. Additionally, the first and second printheads can be contained within multiple housings or a single housing. Further, an optional heater can be placed in thermal contact with the substrate. The heater can supply heat to the predetermined pattern such that the metal salt is reduced to metallic metal at a predetermined rate. Alternatively, the substrate can be heated using a heating plate or other heating device subsequent to printing the metal salt and the reducing agent thereon. The above described components can be incorporated into flatbed printers or standard ink-jet printers which have been modified to print on rigid or flexible substrates, such as optical disks or circuit boards. Generally, a modified ink-jet printer would include inserts which securely hold and move such substrates past the ink-jet printheads.

### EXAMPLES

The following examples 1 to 3 may be used in addition to the method of the present invention. The following example 4 illustrates an embodiment of the invention that is presently best known. However, it is to be understood that the following are only exemplary or illustrative of the application of the principles of the present invention. Numerous modifications and alternative compositions, methods, and systems may be devised by those skilled in the art without departing from the scope of the appended claims. Thus, while the present invention has been described above with particularity, the following examples provide further detail in connection with what are presently deemed to be practical embodiments of the invention.

### Example 1

A first solution of electroless catalyst salt is prepared by dissolving 1 to 5 g/L of SnCl₂·2H₂0 in a solution of about 1 mL concentrated HCl (~37%) per liter of solution. This first solution is placed in a first ink-jet printhead. A second solution of electroless catalyst salt is prepared by dissolving 1 g/L of PdCl₂ in solution of about 1 mL concentrated HCl (-37%) per liter of solution. This second solution is placed in a second ink-jet printhead. A solvated metal composition is prepared including the components shown in Table 1.

**Table 1**

| Component | Concentration |
|---|---|
| Pd(NH₃)₄Cl₂·H₂O | 6.6 g/L |
| Na₂EDTA | 40 ± 5 g/L (up to 70 g/L) |
| NH₄OH (~28%) | 200 mUL (~ pH of 10) |

A glass substrate is heated and maintained at about 60°C using a heating plate. The metal composition is then placed in a third ink-jet printhead. A reducing agent composition of 0.0065 ± 0.0010 M hydrazine is also placed in a fourth ink-jet printhead. The first solution is printed in a simple circuit pattern on the glass substrate. After about 30 seconds the substrate is rinsed with deionized water to remove excess Sn²⁺ ions. Of course, rinsing would be unnecessary if the concentration and amount of tin salt was such that the substrate surface was completely covered with Sn²⁺ ions without excess. The second solution is then printed on the same areas. After about 30 seconds, the substrate is rinsed with water and the metal solution printed on the same pattern. Immediately following, the reducing agent solution is printed on the pattern and the substrate to produce a film initially 150 µm in depth and 2 mm width. The substrate having the compositions printed thereon is allowed to sit for 1 minute. The substrate is then rinsed with water and a palladium metal trace of 35 nm depth is formed. The deposited palladium has a density about 80% that of the bulk density. A film of about 0.1 µm can be produced by three repetitions of ink-jetting of metal solution and reducing agent.

### Example 2

First and second solutions of electroless catalyst salt are prepared and placed in printheads as in Example 1. A solvated metal composition is prepared including the components shown in Table 2.

**Table 2**

| Component | Concentration |
|---|---|
| PdCl₂ | 0.38 g/L |
| NH₄Cl | 4.5 g/L |
| NH₄OH (~28%) | 40 mL/L |

A polyimide substrate is heated to and maintained at about 70 °C. The metal composition is then placed in a third piezoelectric ink-jet printhead. A reducing agent composition of 0.05% aqueous hydrazine is also placed in a fourth piezoelectric ink-jet printhead. The first solution is printed in a simple circuit pattern on the polyimide substrate. After 1 minute, the substrate is rinsed with water and the second solution is then printed on the same areas. After about 30 seconds, the substrate is again rinsed with water and dried. The metal solution is then printed on the same pattern. Immediately following, the reducing agent solution is printed on the pattern and the substrate is allowed to sit for 1 minutes. The substrate is then rinsed and a palladium metal trace of 6 nm depth and 2 mm width is formed.

### Example 3

An electroless active layer is formed by physical vapor deposition of palladium on a silicon substrate. A solvated metal composition is prepared including the components shown in Table 3.

**Table 3**

| Component | Concentration |
|---|---|
| AgNO₃ | 7.5 g/L |
| Na₂EDTA | 50 ± 10 g/L |
| NH₄OH (~28%) | 200 mL/L (to a pH of about 10) |
| 2-pyrrolidone | 100 mL/L |
| Isopropyl alcohol | 200 mL/L |

The silicon substrate is heated to and maintained at about 45 °C. The metal composition is then placed in a first thermal ink-jet printhead. A reducing agent composition having 0.01 M of hydrazine is also placed in a second thermal ink-jet printhead. The metal solution is printed in a circuit pattern having about 1 mm trace width. Immediately following, the reducing agent solution is printed on the pattern and the substrate is allowed to sit for 1 minute. The substrate is then rinsed and a silver metal trace of 70 nm depth is formed.

### Example 4

An electroless active layer is formed by scratching a glass substrate using a diamond needle to form a circuit pattern. A solvate metal composition is prepared including the composition shown in Table 4.

**Table 4**

| Component | Concentration |
|---|---|
| Cu(NO₃)₂·3H₂O | 16 g/L |
| Na₂EDTA | 35± g/L |
| (-C₅H₄N)₂ | 20 mg/L |
| K₄[Fe(CN₆]·3H₂O | 50 mg/L |

The epoxy substrate is heated and maintained at about 55 °C. The metal composition is then placed in a first ink-jet printhead. A reducing agent composition of 35% aqueous formaldehyde is also placed in a second ink-jet printhead. The metal solution is printed on the scratched circuit pattern. Immediately following, the reducing agent solution is printed on the pattern and the substrate is allowed to sit for 2 minutes. The substrate is then rinsed and a copper metal trace of 70 nm depth is achieved.

It is to be understood that the above-referenced arrangements are illustrative of the application for the principles of the present invention. Numerous modifications and alternative arrangements can be devised without departing from the scope of the present invention as set forth in the claims.

## Claims

1. A method of forming metal patterns on a substrate, comprising:
forming an electroless active layer with a pattern by marring the substrate in the pattern;
ink-jetting a metal composition on the pattern, said metal composition including a metal salt; and
ink-jetting a reducing agent composition on the pattern, said reducing agent composition including a reducing agent,
wherein the reducing agent contacts the metal composition and reacts with the metal salt to form a reduced metal.

2. The method of claim 1, wherein the metal of the metal salt is selected from the group consisting of palladium, copper, silver, gold, nickel, cobalt, platinum, rhodium, and mixtures or alloys thereof.

3. The method of claim 2, wherein the metal composition further comprises a metal salt of palladium.

4. The method of claim 1, wherein the reducing agent comprises a member selected from the group consisting of formaldehyde, hydrazine, sodium hypophosphite, sodium boron hydride, dimethylaminoboran, sodium L-ascorbic acid, and mixtures thereof.

5. The method of claim 1, further comprising heating the metal composition and reducing agent compositions on the pattern, wherein the heating is performed at a temperature from 20 °C to 90 °C.

6. The method of claim 1, further comprising the step of forming multiple layers of reduced metal by repeating the ink-jetting of metal composition and reducing agent composition such that the reduced metal has a predetermined depth.

7. The method of claim 1, wherein the reducing agent is ink-jetted on the pattern in an offset area with respect to the metal composition, wherein a portion of each of the metal composition and reducing agent composition are not ink-jetted on the same portions of the pattern.

8. The method of any of claims 1 to 7, wherein said pattern is a circuit pattern.

9. A system for forming metal patterns on a substrate, comprising:
a) an activation system configured to form an electroless active layer with a pattern on the substrate by marring the substrate in the pattern;
b) a first printhead having a first firing chamber reservoir containing an ink-jettable metal composition, said ink-jettable metal composition including a metal salt; and
c) a second printhead having a second firing chamber reservoir containing an ink-jettable reducing agent composition, said ink-jettable reducing agent composition including a reducing agent.

10. The system of claim 9, wherein the metal salt is a palladium salt.

11. The system of claim 9, wherein the activation system includes a third printhead having a third firing chamber reservoir containing an ink-jettable electroless catalyst, said electroless catalyst comprising a member selected from the group consisting of palladium, aluminum protected copper, silver, and mixtures thereof.

12. The system of claim 9, wherein the activation system includes a tip configured for marring the surface in said pattern.

13. The system of claim 9, wherein the substrate further includes a thin hydrophobic layer deposited thereon.

## Patentansprüche

1. Ein Verfahren zum Bilden von Metallstrukturen auf einem Substrat, das folgende Schritte aufweist:
Bilden einer stromlosen aktiven Schicht mit einer Struktur, indem das Substrat in der Struktur beeinträchtigt wird;
Aufbringen, mittels Tintenstrahlen, einer Metallzusammensetzung auf die Struktur, wobei die Metallzusammensetzung ein Metallsalz umfasst; und
Aufbringen, mittels Tintenstrahlen, einer Reduktionsmittelzusammensetzung auf die Struktur, wobei die Reduktionsmittelzusammensetzung ein Reduktionsmittel umfasst,
wobei das Reduktionsmittel die Metallzusammensetzung berührt und mit dem Metallsalz reagiert, um ein reduziertes Metall zu bilden.

2. Das Verfahren gemäß Anspruch 1, bei dem das Metall des Metallsalzes aus der Gruppe ausgewählt ist, die aus Palladium, Kupfer, Silber, Gold, Nickel, Kobalt, Platin, Rhodium und Gemischen oder Legierungen derselben besteht.

3. Das Verfahren gemäß Anspruch 2, bei dem die Metallzusammensetzung ferner ein Palladium-Metallsalz aufweist.

4. Das Verfahren gemäß Anspruch 1, bei dem das Reduktionsmittel ein Element aufweist, das aus der Gruppe ausgewählt ist, die aus Formaldehyd, Hydrazin, Natriumhypophosphit, Natriumborhydrid, Dimethylaminboran, Natrium-L-Ascorbinsäure und Gemischen derselben besteht.

5. Das Verfahren gemäß Anspruch 1, das ferner ein Erhitzen der Metallzusammensetzung und der Reduktionsmittelzusammensetzungen auf der Struktur umfasst, wobei das Erhitzen bei einer Temperatur von 20°C bis 90°C durchgeführt wird.

6. Das Verfahren gemäß Anspruch 1, das ferner den Schritt des Bildens mehrerer Schichten eines reduzierten Metalls durch Wiederholen des Aufbringen, mittels Tintenstrahlen, der Metallzusammensetzung und der Reduktionsmittelzusammensetzung derart, dass das reduzierte Metall eine vorbestimmte Tiefe aufweist, umfasst.

7. Das Verfahren gemäß Anspruch 1, bei dem das Reduktionsmittel in einem versetzten Bereich bezüglich der Metallzusammensetzung mittels Tintenstrahlen auf die Struktur aufgebracht wird, wobei ein Teil sowohl der Metallzusammensetzung als auch der Reduktionsmittelzusammensetzung nicht auf dieselben Abschnitte auf der Struktur mittels Tintenstrahlen aufgebracht wird.

8. Das Verfahren gemäß einem der Ansprüche 1 bis 7, bei dem die Struktur eine Schaltungsstruktur ist.

9. Ein System zum Bilden von Metallstrukturen auf einem Substrat, wobei das System folgende Merkmale aufweist:
a) ein Aktivierungssystem, das dazu konfiguriert ist, eine stromlose aktive Schicht mit einer Struktur auf dem Substrat zu bilden, indem das Substrat in der Struktur beeinträchtigt wird;
b) einen ersten Druckkopf, der ein erstes Abfeuerungskammerreservoir aufweist, das eine mittels Tintenstrahlen aufbringbare Metallzusammensetzung enthält, wobei die mittels Tintenstrahlen aufbringbare Metallzusammensetzung ein Metallsalz umfasst; und
c) einen zweiten Druckkopf, der ein zweites Abfeuerungskammerreservoir aufweist, das eine mittels Tintenstrahlen aufbringbare Reduktionsmittelzusammensetzung enthält, wobei die mittels Tintenstrahlen aufbringbare Reduktionsmittelzusammensetzung ein Reduktionsmittel umfasst.

10. Das System gemäß Anspruch 9, bei dem das Metallsalz ein Palladiumsalz ist.

11. Das System gemäß Anspruch 9, bei dem das Aktivierungssystem einen dritten Druckkopf umfasst, der ein drittes Abfeuerungskammerreservoir aufweist, das einen mittels Tintenstrahlen aufbringbaren stromlosen Katalysator enthält, wobei der stromlose Katalysator ein Element aufweist, das aus der Gruppe ausgewählt ist, die aus Palladium, mit Aluminium geschütztem Kupfer, Silber und Gemischen derselben besteht.

12. Das System gemäß Anspruch 9, bei dem das Aktivierungssystem eine Spitze aufweist, die zum Beschädigen der Oberfläche in der Struktur konfiguriert ist.

13. Das System gemäß Anspruch 9, bei dem das Substrat ferner eine auf dasselbe aufgebrachte dünne hydrophobe Schicht umfasst.

## Revendications

1. Procédé de formation de motifs métalliques sur un substrat, comprenant :
la formation d'une couche active autocatalytique, ayant un motif formé par le marquage du substrat selon le motif ;
le dépôt d'une composition de métal sur le motif au moyen d'une technologie à jet d'encre, ladite composition de métal contenant un sel métallique ; et
le dépôt d'une composition d'agent réducteur sur le motif au moyen d'une technologie à jet d'encre, ladite composition d'agent réducteur contenant un agent réducteur,
dans lequel l'agent réducteur entre en contact avec la composition métallique et réagit avec le sel métallique pour former un métal réduit.

2. Procédé selon la revendication 1, dans lequel le métal du sel métallique est choisi dans le groupe constitué du palladium, du cuivre, de l'argent, de l'or, du nickel, du cobalt, du platine, du rhodium et de leurs mélanges et de leurs alliages.

3. Procédé selon la revendication 2, dans lequel la composition de métal comprend en outre un sel métallique de palladium.

4. Procédé selon la revendication 1, dans lequel l'agent réducteur comprend un élément choisi dans le groupe constitué du formaldéhyde, de l'hydrazine, de l'hypophosphite de sodium, de l'hydrure de sodium et de bore, du diméthylaminoborane, de l'acide L-ascorbique sodique et de leurs mélanges.

5. Procédé selon la revendication 1, comprenant en outre le chauffage de la composition de métal et des compositions d'agent réducteur sur le motif, dans lequel le chauffage est réalisé à une température de 20 °C à 90 °C.

6. Procédé selon la revendication 1, comprenant en outre l'étape de formation de multiples couches de métal réduit par la répétition du dépôt de la composition de métal et du dépôt de la composition d'agent réducteur au moyen d'une technologie à jet d'encre de manière à ce que le métal réduit présente une profondeur prédéterminée.

7. Procédé selon la revendication 1, dans lequel l'agent réducteur est déposé au moyen d'une technologie à jet d'encre sur le motif dans une zone décalée par rapport à la composition de métal, dans lequel le dépôt au moyen d'une technologie à jet d'encre d'une partie de la composition de métal et d'une partie de la composition d'agent réducteur n'est pas effectué sur les mêmes parties du motif.

8. Procédé selon l'une quelconque des revendications 1 à 7, dans lequel ledit motif est un motif de circuit.

9. Système de formation de motifs métalliques sur un substrat, comprenant :
a) un système d'activation conçu pour former une couche active autocatalytique ayant un motif sur le substrat par le marquage du substrat selon le motif ;
b) une première tête d'impression dotée d'un premier réservoir de chambre d'éjection contenant une composition de métal pouvant être déposée au moyen d'une technologie à jet d'encre, ladite composition de métal pouvant être déposée au moyen d'une technologie à jet d'encre contenant un sel métallique ; et
c) une deuxième tête d'impression dotée d'un deuxième réservoir de chambre d'éjection contenant une composition d'agent réducteur pouvant être déposée au moyen d'une technologie à jet d'encre, ladite composition d'agent réducteur pouvant être déposée au moyen d'une technologie à jet d'encre contenant un agent réducteur.

10. Système selon la revendication 9, dans lequel le sel métallique est un sel de palladium.

11. Système selon la revendication 9, dans lequel le système d'activation comprend une troisième tête d'impression dotée d'un troisième réservoir de chambre d'éjection contenant un catalyseur autocatalytique pouvant être déposé au moyen d'une technologie à jet d'encre, ledit catalyseur autocatalytique comprenant un élément choisi dans le groupe constitué du palladium, du cuivre protégé par de l'aluminium, de l'argent et de leurs mélanges.

12. Système selon la revendication 9, dans lequel le système d'activation comprend une pointe conçue pour marquer la surface selon ledit motif.

13. Système selon la revendication 9, dans lequel le substrat comprend en outre une couche mince hydrophobe sur celui-ci.
